# EUROPEAN PATENT APPLICATION

(11) **EP 2 088 157 A1**
(43) Date of publication of application: **12.08.2009**
(21) Application number: 07831926.6
(22) Date of filing: 15.11.2007
(51) Int. Cl.: C07K 14/78, A23L 1/30, A23L 1/305, A23L 2/52, A61K 35/60, A61K 38/00, A61K 38/17, A61P 3/02, A61P 17/16, A61P 19/08, A61P 19/10, A61P 43/00, C07K 2/00

(54) **COLLAGEN PEPTIDE COMPOSITION AND FOOD OR BEVERAGE CONTAINING THE SAME**

(30) Priority: 15.11.2006 JP 2006309032
(71) Applicant: Meiji Seika Kaisha Ltd., Tokyo 104-8002 (JP)
(72) Inventor: MATSUMOTO, Hitoshi, Sakado-shi Saitama 350-0289 (JP); OHARA, Hiroki, Sakado-shi Saitama 350-0289 (JP); NAKAJIMA, Takanori, Kitakatsuragi-gun Nara 635-0833 (JP); SUGIHARA, Fumihito, Kishiwada-shi Osaka 596-0812 (JP); TAKASAKI, Hajime, Kitakatsuragi-gun Nara 636-0002 (JP)
(74) Representative: Gillard, Richard Edward
(86) International application number: PCT/JP2007/072196
(87) International publication number: WO 2008/059927

(57) **Abstract**

An object of the present invention is to elucidate a collagen peptide composed of oligopeptides having ability to enter the blood higher than that of conventional collagen peptides and thus to provide a food or beverage mixed with the collagen peptide. The present invention provides a collagen peptide composition obtainable by digesting a collagen or gelatin with protease, which comprises 70% to 100% by weight of peptides with a molecular weight 500 or more to 3000 or less, less than 10% by weight of peptides with a molecular weight of less than 500, and less than 20% by weight of peptides with a molecular weight of more than 3000, based on the total weight of the composition, wherein the ratio of N-terminal glycine residues to total of the N-terminal amino acid residues of the peptides in the composition is 33 mol% or more to 65 mol% or less.

## Description

### Technical Field

The present invention relates to a collagen peptide composition, more specifically a collagen peptide composition composed of oligopeptides having good ability to enter the blood, and to a food or beverage containing the collagen peptide composition.

### Background Art

Collagen is a protein composing dermis, ligament, tendon, bone, cartilage, and the like, which is a major ingredient of the extracellular matrix of multicellular animals. Collagen is present everywhere throughout skin, blood vessels, viscera, bone tissues, and the like and accounts for about 30% of body-composing proteins. In the skin, 70% of dermis is made up of collagen. Fascia wrapping individual muscles is also made up of collagen.

Collagen protein is made of 3 polypeptide chains (α chains) each having a repeated structure of Gly-X-Y (where X and Y are amino acids other than Gly) and a molecular weight of approximately one hundred thousand, wherein the 3 polypeptide chains are assembled to form a helical structure. While a collagen protein has amino acids unique to collagen, such as hydroxyproline and hydroxylysine, it lacks tryptophan, which is an essential amino acid. Thus, its amino acid score is zero and it has historically attracted no attention as a nutrient.

However, in recent years, collagen has been found to exert various physiological effects and pharmacological effects. Collagen, gelatin prepared via heat denaturation of collagen, and collagen peptides that are hydrolysates thereof are widely used as raw materials for cosmetics and foods or biologically functional materials for pharmacological products. The physiological effects or pharmacological effects of collagen that have been revealed to date include osteoblast growth accelerating effects and bone strengthening effects that lead to prevention and/or improvement of osteoporosis (see Patent Documents 1 and 2), effects for accelerating metabolism in the living tissue, which ameliorate the declining functions of living tissues with aging (see Patent Documents 3 and 4), skin metabolism accelerating effects, and skin activating effects (see Patent Documents 4 and 5), for example. However, all such prior art documents merely disclose that active ingredients are collagen, gelatin, or hydrolysates of collagen or gelatin. They do not specifically reveal the molecular weights of such active ingredients or what kinds of amino acid sequences such active ingredients have. In addition, sufficient exertion of the above physiological effects and pharmacological effects has required the high intake of collagen, gelatin, or hydrolysates of either thereof.

Furthermore, Taniguchi et al., have reported that as a result of comparison and examination of a collagen hydrolysates (a peptide mixture or peptides) obtained via treatment with collagenase and an amino acid mixture having the same rate as the product in terms of effects of accelerating skin collagen synthesis in rats, the amino acid mixture has been observed to exert no effects and only the products digested with collagenase have been observed to exert effects (Non-Patent Document 1). These results suggest that physiological effects and pharmacological effects of the products digested with collagenase are not exerted by reconstituted protein from amino acids absorbed into the body, but by peptides that have entered the blood. However, this report fails to clarify the kinds of amino acid sequences that effective peptides have.

Meanwhile, Patent Document 6 discloses a biological collagen synthesis accelerator containing a tripeptide of an amino acid sequence of Gly-X-Y as a collagen hydrolysate product obtainable by digestion with collagenase. However, samples for the collagen accelerating activity test in Patent Document 6 have not been administered via oral administration, but via gastric tubes. Moreover, the ability to enter the blood of orally-ingested peptides has never been examined in such document. Furthermore, collagenase, which is an enzyme for preparation of the tripeptide, is not included in the list of food additives of the Ministry of Health, Labour and Welfare. The safety of collagenase is unconfirmed, so that it is actually difficult to use this peptide for foods.

Furthermore, similarly to the above Patent Document, Patent Document 7 discloses a collagen acceleration activator containing various tripeptides represented by Gly-X-Y. Patent Document 7 reports that the above tripeptides are more rapidly and efficiently digested and absorbed than conventional collagen, gelatin, hydrolysates thereof, and free amino acids. In this Patent Document, an *in vivo* kinetic test has been conducted for tripeptides labeled with radioisotopes, but it has never elucidated the issue of whether the main body of radioactivity, which is distributed in plasma or various tissues, is an unmetabolized form of tripeptide, a dipeptide, or a free amino acid. Also, an enzyme for preparation of a tripeptide is similarly collagenase. Thus, in terms of safety, it is actually difficult to use collagenase for foods. Also, all peptides composing a tripeptide mixture, which is contained in a collagen acceleration activator disclosed in this Patent Document, are peptides of which the N-terminal amino acid residue is glycine (Gly-X-Y). Moreover, the Patent Document has never described adjustment of the ratio of glycine in the N-terminal amino acid residues of the tripeptides in the mixture.

Furthermore, Patent Document 8 discloses that a collagen peptide composition rich in peptides with a molecular weight ranging from 400 to 3000 exerts excellent usability and skin permeability when mixed with skin cosmetics or pharmacological products. However, this document discloses neither evaluation of the ability to enter the blood in the case of oral intake nor adjustment of the ratio of glycine in the N-terminal amino acid residues of peptides in the composition.
Patent Document 1: JP Patent Publication (Kokai) No. 9-255588 A (1997)
Patent Document 2: JP Patent Publication (Kokai) No. 11-12192 A (1999)
Patent Document 3: JP Patent Publication (Kokai) No. 7-278012 A (1995)
Patent Document 4: JP Patent Publication (Kokai) No. 9-67262 A (1997)
Patent Document 5: JP Patent Publication (Kokai) No. 2000-201649 A
Patent Document 6: JP Patent Publication (Kokai) No. 2001-131084 A
Patent Document 7: JP Patent Publication (Kokai) No. 2003-137807 A
Patent Document 8: JP Patent Publication (Kokai) No. 2006-151847 A
Non-Patent Document 1: Meeting of the Japanese Society of the Veterinary Science, Lecture Summary, Vol. 13, 2nd, Page 126, PS-5014 (2001. 09. 07)

### Disclosure of the Invention

### Problems to be solved by the Invention

As described above, conventionally the sufficient *in vivo* exertion of the physiological effects and pharmacological effects of collagen peptides require intake of as much as 5 g to 10 g thereof. However, long-term high intake of only a specific protein in addition to general meals is difficult and is unfavorable in terms of nutritional science. Also, it is important to cause a collagen peptide to enter the blood not in the form of an amino acid but in the form of an oligopeptide for *in vivo* exertion of physiological effects and pharmacological effects of the collagen peptide after oral intake thereof.

Therefore, an object of the present invention is to elucidate a collagen peptide composed of oligopeptides with ability to enter the blood higher than that of conventional collagen peptides and thus to provide a food or beverage mixed with the collagen peptide.

### Means for solving the problems

As a result of intensive studies to achieve the above object, the present inventors have elucidated that a collagen peptide composition having a specific molecular weight distribution and characterized by ratio of N-terminal glycine residues to total of the N-terminal amino acid residues of the peptides in the composition that is within a specific range is excellent in ability to enter the blood when orally ingested. Thus, the present inventors have completed the present invention.

The present invention encompasses the following (1) to (4).
(1) A collagen peptide composition obtainable by digesting a collagen or gelatin with protease, which comprises 70% to 100% by weight of peptides with a molecular weight 500 or more to 3000 or less, less than 10% by weight of peptides with a molecular weight of less than 500, and less than 20% by weight of peptides with a molecular weight of more than 3000, based on the total weight of the composition, wherein the ratio of N-terminal glycine residues to total of the N-terminal amino acid residues of the peptides in the composition is 33 mol% or more to 65 mol% or less.
(2) The collagen peptide composition according to (1), wherein the protease is serine protease or cysteine protease.
(3) The collagen peptide composition according to (1) or (2), wherein the collagen or the gelatin is derived from fishes.
(4) A food or beverage, which contains the collagen peptide composition according to any one of (1) to (3).

The present invention is explained in detail as follows. This application claims priority of Japanese patent application No. 2006-309032 filed on November 15, 2006, and encompasses the content described in the description of the above patent application.

### Effect of the Invention

According to the present invention, a collagen peptide composition is provided that is composed of oligopeptides having ability to enter the blood higher than that of conventional collagen peptides. Accordingly, a food or beverage is mixed with the collagen peptide composition of the present invention and is then orally ingested, so that the physiological effects and the pharmacological effects can be exerted efficiently in an amount smaller than that of conventional products.

### Brief Description of the Drawings

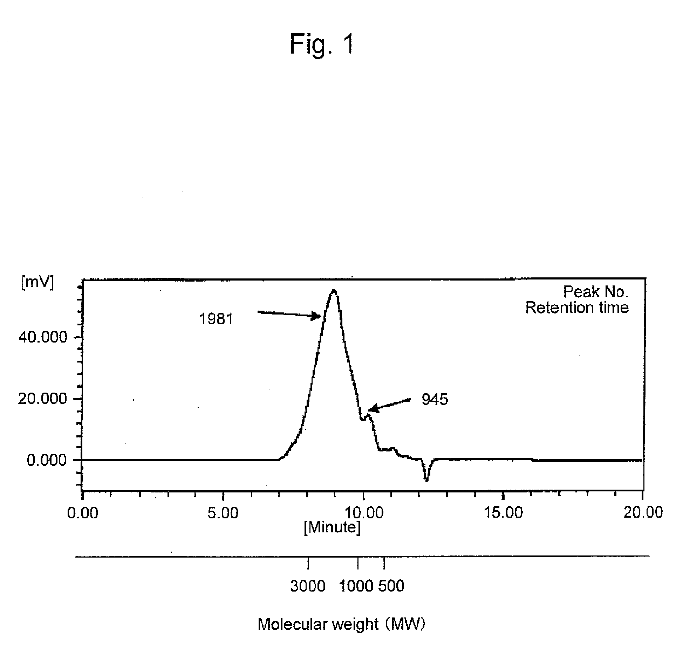

Fig. 1 shows the molecular weight distribution of a collagen peptide composition of the present invention.

### Best Mode for Carrying Out the Invention

The present invention is described in detail as follows.

### 1. Collagen peptide composition

The collagen peptide composition of the present invention is **characterized in that** the peptides in the composition have a specific molecular weight distribution and that the ratio of N-terminal glycine residues to total of the N-terminal amino acid residues of the peptides in the composition is within a specific range.

The above molecular weight distribution of the peptides in the composition can be measured by a conventional method. For example, an HPLC (High Performance Liquid Chromatography) method using a gel filtration column is employed. It is prefererable that the molecular weight distribution forms a curve (like a mountain) with the peak at the molecular weight of approximately 2000. In addition, it is prefererable that the molecular weight distribution comprises 70% to 100% by weight of peptides with a molecular weight 500 or more to 3000 or less, less than 10% by weight of peptides with a molecular weight of less than 500, and less than 20% by weight of peptides with a molecular weight of more than 3000, based on the total weight of the composition. The peptides with a molecular weight of higher than 3000 are not desiareble in terms of poor digestion and absorption. Meanwhile, the peptides with lower molecular weights are generally desirable in terms of easiness of digestion and absorption. However, it is undesirable for too many peptides with a molecular weight of lower than 500 to be present, since such peptides are digested into amino acids in the step of digestion, leading to even poorer ability to enter the blood of oligopeptides.

Moreover, the ratio of N-terminal glycine residues to total of the N-terminal amino acid residues of the total peptides in the composition is preferably 33 mol% or more to 65 mol% or less, and more preferably 35 mol% or more to 65 mol% or less. Here the expression "the ratio of N-terminal glycine residues to total of the N-terminal amino acid residues" refers to a value found by analyzing the first amino acid residue from the N-terminus of each peptide in the collagen peptide composition and then expressing the ratio of N-terminal glycine residues to the thus detected total of the N-terminal amino acid residues in terms of molar percentage (mol%). N-terminal amino acid residues may be analyzed using an amino acid sequence analyzer with which an Edman method is carried out in an automated manner.

The collagen peptide composition of the present invention is further **characterized in that** oligopeptides have high ability to enter the blood. Here, examples of such "oligopeptides" include dimers such as prolyl-hydroxyproline, isoleucyl-hydroxyproline, leucyl-hydroxyproline, and phenylalanyl-hydroxyproline and trimers such as alanyl-hydroxyprolyl-glycine and seryl-hydroxyprolyl-glycine.

The collagen peptide composition of the present invention is **characterized in that** the level of hydroxyproline-containing peptide (that is released into blood 1 to 2 hours after a general adult orally ingests 25 g of the collagen peptide composition) ranges from approximately 70 nmol/ml to 170 nmol/ml.

The term "collagen peptide composition" (hereinafter, may also be simply referred to as "collagen peptide" in this description) to be used in the present invention refers to a mixture of peptides that are obtained by hydrolysis of collagen or gelatin.

Collagen or gelatin that is used as a raw material for the "collagen peptide composition" to be used in the present invention is not limited and may be any collagen or gelatin derived from a mammal such as cattle or swine or the same derived from fishes such as shark. However, collagen or gelatin derived from fishes having relatively weak collagen odor is preferred since it is mixed with a food or beverage. Particularly preferable raw materials are fish scales. Examples of fish scales are not particularly limited by fish species or the sites of scales, as long as they are fish scales containing collagen tissues. Examples of fish species from which fish scales are derived include tilapia (fish of the family *Cichlidcae*), Nile perch (*Lates niloticus*), common carp (*Cyprinus carpio*), grass carp (*Ctenopharyngodon idellus*), red snapper (*Lutjanus sebae*), golden threadfin bream (*Nemipterus virgatus*), parrotfish (fish of the family *Scaridae*), sardine (fish related to herrings, the family *Clupeidae*), star snapper (*Lutjanus stellatus*), lizardfish or snakefish (fish of the family *Synodontidae*), grass carp (*Ctenopharyngodon idellus*), cod (fish of the family *Gadidae*), black carp (*Mylopharyngodon piceus*), silver carp (*Hypophthalmichthys molitrix*), and bighead carp (*Aristichthys nobilis*).

Collagen can be obtained from the above mammals' bone or leather portions or fish bone, fishskin, fish scale portions, or the like. Various materials such as bone may be subjected to conventionally known treatment such as delipidization or extraction. Prior to use fish scales, a step of washing (e.g., washing with water) is preferably carried out in advance for several times to remove dirt or contaminants adhered on fish scales, delipidization is carried out to remove fat and oil contents, and then decalcification is carried out to remove inorganic materials such as phosphorus and calcium.

Also, when collagen is used as a raw material, collagen is preferably gelatinized once in terms of easier control of the ratio of N-terminal glycine residues to total of the N-terminal amino acid residues. Gelatin is prepared by heat-denaturing and solubilizing collagen. Gelatinization is carried out by pretreating a collagen raw material with acid or alkali, followed by heating and extraction. Either acid treatment or alkaline treatment may be carried out as pretreatment according to the type of a collagen raw material. Acid treatment is carried out by immersing a collagen raw material in inorganic acid such as hydrochloric acid or sulfuric acid for 5 to 20 days. Alkaline treatment is carried out by immersing a collagen raw material in a lime solution or the like containing hydrated lime (calcium hydroxide) for 2 to 3 months, for example. Moreover, generally a pretreated raw material is washed with water to remove excessive acid or alkali, subjected to 1st extraction with hot water at 50°C to 60°C and then subjected to 2nd extraction with hot water at a temperature higher than that of the 1 st extraction.

### 2. Production of collagen peptide composition

A "collagen peptide composition" to be used in the present invention is produced as described below, for example. Protease treatment was carried out for collagen or gelatin obtained from collagen by the above treatment so as to digest the collagen molecules into the form of peptide. As protease to be used herein, serine protease or cysteine protease is preferably used so that the molecular weight distribution of the collagen peptide composition and the ratio of N-terminal glycine residues to total of the N-terminal amino acid residues are each within the range as predetermined above. Collagenase is undesirable since most (95% or more) of the N-terminal amino acid residues are glycines (see Sakai et al., Fragrance Journal, March 2006, pp. 54-60). Examples of serine protease include subtilisin, thermitase, proteinase K, lantibiotic peptidase, kexin, cucumisin, trypsin, chymotrypsin, thrombin, kexin, furin, Xa factor, and elastase. Examples of cysteine protease include actinidain and bromelain. One type of them may be used or a plurality of enzymes may be used in combination.

Enzyme treatment is carried out by, for example, reacting 0.02 parts by weight to 5 parts by weight of enzyme with 100 parts by weight of collagen or gelatin at 30°C to 70°C for 0.5 to 24 hours. The above temperature and time for treatment are just examples and may be adequately adjusted to enable sufficient exertion of enzyme functions and to avoid too much progression of collagen digestion, in order to obtain a collagen peptide composition having a target molecular weight distribution and a target composition of N-terminal amino acids.

After the above enzyme treatment, the resultant is heat-treated at 70°C to 100°C, so as to inactivate the enzyme. Excessive high-temperature treatment is undesirable since such treatment may destroy the flavor.

At the stage after completion of the above enzyme treatment, a collagen peptide composition is in a state of being dissolved or dispersed in an enzyme treatment solution. The collagen peptide composition can be purified from the enzyme solution by various generally employed purification means. Such purification means are not particularly limited. For example, improvement of color tone and flavor and removal of impurities can be very conveniently carried out by the addition of activated carbon. Impurities can also be removed by conventionally known solid-liquid separation such as filtration or centrifugation. A collagen peptide solution treated as described above is dried by a method such as spray drying or using a drum dryer, so that powderization can be carried out.

### 3. Food or beverage containing collagen peptide composition

The collagen peptide composition is **characterized in that** the oligopeptides have good ability to enter the blood, so that it can be provided as a food or beverage for daily intake. Examples of the forms of the collagen peptide composition in foods or beverages include a form such that the collagen peptide composition is directly a food or beverage and a form such that the collagen peptide composition is a raw material or an intermediate product upon production of a food or beverage.

In the present invention, the term "food(s) or beverage(s)" is used to include health foods, functional foods, foods for specified health use, foods for sick or injured persons, and the like. Moreover, when such food or beverage of the present invention is used for mammals other than humans, the term can be used to include a feedstuff.

The form of a food or beverage to be mixed with the collagen peptide composition may be either a solid form or a liquid form. Specific examples of the types of foods or beverages include, but are not limited to, beverages such as soft drinks, carbonated drinks, nutritional beverages, fruit beverages, and milk beverages (including a concentrated stock solution of such a beverage and a dry powder for preparation of such a beverage); frozen desserts such as ice cream, ice sherbet, and shaved ice; noodles such as buckwheat noodles, wheat noodles, bean-starch vermicelli, *gyoza* wraps (pot stickers), *su my* wraps (dim sum), Chinese noodles, and instant noodles; confectioneries such as *ame* (candy), chewing gum, candy, gummi candy, gum, caramel, chocolate, tablet sweets, snacks, baked goods (e.g., biscuit), jelly, jam, and cream; fish-livestock processed foods such as minced and steamed fish, hamburger, ham, and sausage; dairy products such as processed milk, fermented milk, yogurt, butter, and cheese; fats and oils and fat and oil processed foods such as salad oil, tempura oil, margarine, mayonnaise, shortening, whipped cream, and dressing; seasonings such as sauce and baste; and soup, stew, curry, bread, jam, salad, daily dishes, and Japanese pickles.

The food or beverage of the present invention can also be mixed with ingredients other than the above collagen peptide composition. For example, water soluble collagen, gelatin, and the like each having a molecular weight larger than that of a peptide can be combined. It is expected that functions and features that are unable to obtain by the use of the collagen peptide composition alone can be exerted through combination of a plurality of collagen ingredients. Moreover, particularly, cock's comb extract containing hyaluronic acid, a bovine, swine, or human placental extract, bovine or swine elastin and a hydrolysate thereof (obtainable by treatement with acid, alkali, and enzyme or the like) or a water soluble elastin derivative thereof, keratin and a hydrolysate thereof or a derivative thereof, a silk protein and a hydrolysate thereof or a derivative thereof, a hydrolysate of swine or bovine hemocyte protein (globin peptide), a decomposed product of bovine or swine hemoglobin (e.g., hemin, hematin, heme, protoheme, and heme iron), milk, casein and a hydrolysate thereof or a derivative thereof, a fat-free milk powder and a hydrolysate thereof or a derivative thereof, lactoferrin and a hydrolysate thereof, a hen egg ingredient, a decomposed product of a fish food, a nucleic acid-related substance (e.g., ribonucleic acid and deoxyribonucleic acid), or the like can also be added.

The food or beverage of the present invention may be adequately mixed with a generally used additive according to the type of a food or beverage. Examples of an additive include sweeteners such as sugar, fructose, isomerized sugar syrup, glucose, aspartame, and stevia, acidulants such as citric acid, malic acid, and tartaric acid, excipients such as dextrin and starch, binders, diluents, flavoring agents, buffering agents, thickeners, gelatinizing agents, colorants, stabilizers, emulsifiers, dispersants, suspending agents, and antiseptics.

The amount of the collagen peptide composition to be mixed with the food or beverage of the present invention may be any amount that allows the physiological effects and/or pharmacological effects to be exerted. In view of the general intake level of a target food or beverage, the intake level per day for an adult generally ranges from 100 mg to 10,000 mg and preferably ranges from 1,000 mg to 6,000 mg. For example, in the case of a food in a solid form, the intake level ranges from 10 % to 50 % by weight and in the case of a liquid food such as a beverage, the intake level preferably ranges from 1 % to 10 % by weight.

Typical examples of foods or beverages for mixing are listed specifically as follows, but the examples are not limited thereto.

Fruit juice beverage: collagen peptide composition (0.5 to 30 parts by weight), fruit juice (1 to 50 parts by weight), isomerized sugar syrup (5 to 20 parts by weight), acidulant (e.g., citric acid) (0.01 to 1.0 parts by weight), flavoring agent (0.1 to 1.0 parts by weight), and water (30 to 95 parts by weight).

Fruit jelly·jelly beverage: collagen peptide composition (0.5 to 20 parts by weight), fruit juice (1 to 40 parts by weight), granulated sugar (5 to 20 parts by weight), acidulant (e.g., citric acid) (0.01 to 1.0 parts by weight), gelatinizing agent (e.g., gelatin) (0.5 to 10.0 parts by weight), flavoring agent (0.1 to 1.0 parts by weight), and water (15 to 95 parts by weight).

Powdered food: collagen peptide composition (0.5 to 80 parts by weight), maltodextrin (5 to 20 parts by weight), thickener (e.g., gelatin) (0.1 to 5.0 parts by weight), emulsifier (e.g., sugar ester) (0.1 to 5.0 parts by weight), and sweetener (e.g., aspartame) (0.01 to 1 parts by weight).

Food in the form of tablet: Powders containing a combination of a collagen peptide composition (0.5 to 80 parts by weight), maltodextrin (5 to 20 parts by weight), a thickener (e.g., gelatin) (0.1 to 5.0 parts by weight), an emulsifier (e.g., sugar ester) (0.1 to 5.0 parts by weight), and a sweetener (e.g., aspartame) (0.01 to 1 parts by weight) are tabletted.

Various physiological effects and pharmacological effects are exerted through the oral intake of the food or beverage of the present invention, such as the curing of joint diseases (e.g., osteoarthritis and chronic rheumatism), the alleviation of osteoporosis, the prevention of arteriosclerosis and hypertension, the accelerated curing of wound sites, the curing of dermatological diseases (eczema, skin roughness, atopic dermatitis, and pigment deposition), the improvement of moisture-retaining properties of skin, the improvement of skin aging (e.g., wrinkles, pigmented spots, dullness, sag, and keratinization), the prevention of hair aging (e.g., gray hair, hair loss, and thinning hair), and antiulcer effects.

### Examples

Hereafter, the present invention is described in greater detail with reference to the following examples, although the present invention is not limited to these examples.

### (Example 1) Preparation of the collagen peptide composition of the present invention

Fish scale gelatin (1.0 kg) (Nitta Gelatin Inc.) treated with acid was dissolved in 2.0 kg of hot water at 75°C. Subtilisin (5.5 g) (Wako Pure Chemical Industries, Ltd.) was added as protease to the thus obtained gelatin solution, and the pH of the solution was adjusted to 8, followed by 4 hours of enzyme reaction at 50°C. After completion of the reaction, the solution was heated at 90°C or higher so as to inactivate the enzyme. Pulverized activated carbon (20 g) was added, microfiltration was carried out, and then spray drying was carried out. Thus, a powdery collagen peptide composition was obtained.

The average molecular weight of the thus obtained collagen peptide composition was measured by carrying out gel filtration high performance liquid chromatography (GF-HPLC) under the following conditions. Data processing was carried out using Multistation GPC-8020 Software Ver 4.0 (TOSOH). The average molecular weight of the collagen peptide composition was calculated from the average retention time of the same using a calibration curve that had been separately prepared based on the retention time of a molecular weight marker for a molecular weight ranging from 307 to 17800.

### (Analytical conditions)

Column: TSK-GEL 2500PW_{XL} (300 × 7.8 mm)
Eluent: 45% acetonitrile (containing 0.1% trifluoroacetic acid)
Flow rate: 0.8 ml/min
Detection wavelength: 214 nm

Fig. 1 shows the molecular weight distribution of the collagen peptide composition of the present invention. The average molecular weight of the collagen peptide composition was 2000, suggesting that the composition was a mixture of peptides, most of which had a molecular weight of 500 or more to 3000 or less. Details about percentages are as follows. Peptides with molecular weights of 500 or more to 3000 or less accounted for 88.1 % by weight, peptides with molecular weights of more than 3000 accounted for 10.3% by weight, and peptides with molecular weights of less than 500 accounted for 1.6% by weight, based on the weight of the composition.

### (Example 2) Test for comparison of ability to enter the blood (absorption test (1))

The ability to enter the blood of the collagen peptide composition (average molecular weight of 2000) of the present invention prepared in Example 1 was compared with the same of commercially available collagen peptide compositions. As commercially available collagen peptide compositions, fishskin collagen peptide A (Nippi, inc., Trade name: Nippi Peptide FCP; average molecular weight of 5000), fishskin collagen peptide B (MARUHA; Trade name: Fish Collagen WP; average molecular weight of 3000), fish scale collagen peptide C (RABJ; Trade name: Marine Collagen MS5; average molecular weight of 500), and pig skin collagen peptide D (Nitta Gelatin Inc., Trade name: Super Collagen Peptide SCP5000; average molecular weight of 5000) were used.

The test was conducted based on the report of Iwai et al., (Agric. Food Chem., 2005, Vol. 53, No. 16, p 6531-6536). The test conducted herein was a crossover test such that a wash-out period of 6 or more days was set for 5 human volunteers, and each subj ect underwent single instance of intake of all types of collagen peptide composition described above as test samples. After the subjects fasted for 12 hours, blood was collected before intake and then each subject ingested each test sample. The intake level of each test sample was determined to be 25 g/65 kg body weight. At 0, 5, 1, 2, 4, and 7 hours after intake, 5 mL of blood was collected. Blood collection tubes in which blood had been collected were turned upside down for several times for mixing, allowed to stand for approximately 15 minutes in ice, and then subjected to centrifugation (3000 rpm, 15 min, 4°C), so that blood plasma was obtained. Ethanol (900 µL) was added to 300 µL of blood plasma, the mixture was stirred using a Vortex for 15 seconds, and then the mixture was subjected to centrifugation (12,000 rpm, 10 min, 4°C), so that supernatants were obtained. The blood plasma collected from each subject at each blood collection time was cryopreserved at -80°C until the use thereof for analysis.

The levels of hydroxyproline-containing peptide in blood plasma were found by separately measuring the total hydroxyproline levels and the free hydroxyproline levels and then finding the difference between the two. The total hydroxyproline levels were measured according to the method of Sato et al., (Sato K. et al., J. Agric. Food Chem. 1992, 40, 806-810) by carrying out hydrolysis of blood plasma samples with 6N hydrochloric acid, carrying out inducing the hydrolysates to phenylisothiocyanate derivatives using phenylisothiocyanate (PITC), and then carrying out HPLC under the following conditions. Also, the free hydroxyproline levels in blood plasma were measured by carrying out HPLC under the same conditions for each deproteinated blood plasma sample.

### (Analytical conditions)

Column: TSK8OTsQA (250 × 2.0 mm)
Eluent: (solution A) 50 mM sodium acetate buffer (pH 6)
   (solution B) acetonitrile
Elution conditions: solution B 5-10% (0-8 min), solution B 70% (8-11 min), and solution B 5% (11 min)
Flow rate: 0.18 mL/min
Detection wavelength: 254 nm

Table 1 below shows the mean value ± S.E. of AUC₀₋₇ values (AUC: area under the curve of blood concentration of hydroxyproline-containing peptide-time) (hr · nmol/ml), as calculated for each blood plasma sample.

**Table 1**

| Blood hydroxyproline-containing peptide levels after the oral intake of collagen peptide compositions | |
|---|---|
| Collagen peptide composition | Hydroxyproline-containing peptide level (hr•nmol/ml: mean value ± S.E.) |
| Collagen peptide of the present invention | 401.1 ± 29.2 |
| Collagen peptide A | 220.6 ± 18.7^{*} |
| Collagen peptide B | 245.4 ± 18.5^{*} |
| Collagen peptide C | 190.0 ± 11.8^{*} |
| Collagen peptide D | 277.2 ± 12.6^{*} |

| | |
|---|---|
| ^{*}: P < 0.01 | |

Furthermore, blood hydroxyproline-containing peptide levels reach a peak at 1 to 2 hours after intake. Table 2 shows the mean value ± S.E. of the hydroxyproline-containing peptide level (nmol/ml) at 1 hour and the same at 2 hours after intake of each collagen peptide.

**Table 2**

| Blood hydroxyproline-containing peptide lelvels at 1 hour and 2 hours after the oral intake of collagen peptide compositions | | |
|---|---|---|
| Collagen peptide composition | 1 hour later (nmol/ml) | 2 hours later (nmol/ml) |
| Collagen peptide of the present invention | 94.8 ± 9.3 | 125.5 ± 12.9 |
| Collagen peptide A | 53.8 ± 5.7^{*1} | 63.0 ± 5.6^{*1} |
| Collagen peptide B | 65.3 ± 6.5^{*2} | 76.2 ± 6.7^{*2} |
| Collagen peptide C | 55.3 ± 11.8^{*1} | 67.1 ± 10.0^{*1} |
| Collagen peptide D | 57.3 ± 2.5^{*1} | 83.3 ± 6.6^{*1} |

| | | |
|---|---|---|
| ^{*1}: P < 0.05, ^{*2}: P < 0.01 | | |

As shown in Table 1, when AUCs were compared, the ability to enter the blood of the collagen peptide composition of the present invention was significantly increased compared with the cases of the other commercially available collagen peptide compositions. Moreover, as shown in Table 2, ability to enter the blood at 1 hour and the same at 2 hours after the intake of the collagen peptide composition of the present invention had significantly increased 1.4 to 1.8 times and 1.5 to 2.0 times greater than the same values for the other commercially available collagen peptide compositions.

### (Example 3) Composition of peptides in blood after the oral intake of collagen peptide composition

Of blood plasma samples collected in Example 2, blood plasma samples collected after the intake of the collagen peptide composition of the present invention, commercially available collagen peptide A (derived from fishskin), or collagen peptide D (derived from pig skin) were used. The ethanol supernatant fraction of each sample was dried and solidified using a centrifugal vacuum drier, 200 µL of 30% acetonitrile containing 0.1% trifluoroacetic acid was added for dissolution, and then fractionation was carried out by HPLC under the following conditions.

### (Analytical conditions)

Column: Superdex peptide HR10/30 (Amersham Pharmacia, Piscataway, NJ, U.S.A.)
Eluent: 30% acetonitrile (containing 0.1 % trifluoroacetic acid)
Flow rate: 0.5 mL/min
Detection wavelength: 230 nm
Column temperature: room temperature
Analysis time: 60 minutes
Elution samples were collected every minute using a fraction collector. Each fraction having a molecular weight ranging from 200 to 500 obtained by gel filtration chromatography was concentrated as a peptide fraction using a centrifugal vacuum drier to approximately 50 µL. The resultant was then fractionated by reverse phase chromatography under the following conditions.

### (Analytical conditions)

Column: Inertsil ODS-3 (250 mm × 4.6 mm i.d., GL Science, Tokyo)
Eluent: (solution A) 0.1% trifluoroacetic acid
   (solution B) 80% acetonitrile (containing 0.1 % trifluoroacetic acid)
Elution conditions: solution B 0% (0-15 min), solution B 0-50% (15-30 min), solution B 100% (30-40 min), and solution B 0% (40-55 min)
Flow rate: 1 mL/min
Column temperature: 43.0°C
Detection wavelength: 230 nm

Amino acid analysis and protein sequencing were carried out for each peak resulting from fractionation by reverse phase chromatography. The composition of peptides in blood after the oral intake of each collagen peptide composition was calculated. Amino acid analysis was carried out in a manner similar to that in Example 2; that is, according to the method of Sato et al., (Sato K. et al, J. Agric. Food Chem. 1992, 40, 806-810). As standard solutions, a PTH amino acid standard mixture (solution) (Applied Biosystems) and a PTH hydroxyproline preparation (solution) (Applied Biosystems) were used. The protein sequencing was carried out by adding the fraction solution of each peak dropwise to a PVDF (poly(vinylidene fluoride)) membrane and then analyzing the protein sequence using a protein sequencer (Applied Biosystems). Table 3 below shows data obtained at 1 hour after intake, representing typical results.

**Table 3**

| Composition of peptides in blood after the oral intake of the collagen peptide compositions | | | |
|---|---|---|---|
| Peptides | Collagen Peptide of the present invention | Collagen Peptide A | Collagen Peptide D |
| Ala-Hyp-Gly | 30.9 | 0 | 0 |
| Ser-Hyp-Gly | 23.7 | 0 | 0 |
| Pro-Hyp | 69.5 | 22.6 | 54.4 |
| Pro-Hyp-Gly | 4.4 | 1.6 | 0 |
| Ile-Hyp | 3 | 3.8 | 0.6 |
| Leu-Hyp | 10.5 | 14.5 | 1.7 |
| Phe-Hyp | 3.6 | 3.8 | 0.6 |

| | | | |
|---|---|---|---|
| (Numerical value: nmol/ml) | | | |

As shown in Table 3, the composition of peptides in blood after the oral intake of collagen peptide A or D was composed mainly of a dimer of prolyl-hydroxyproline. Dimers such as isoleucyl-hydroxyproline, leucyl-hydroxyproline, and phenylalanyl-hydroxyproline were also observed. On the other hand, in the composition of peptides in blood after the oral intake of the collagen peptide composition of the present invention, in addition to conventional dimers such as prolyl-hydroxyproline, isoleucyl-hydroxyproline, leucyl-hydroxyproline, and phenylalanyl-hydroxyproline, trimers such as alanyl-hydroxyprolyl-glycine and seryl-hydroxyprolyl-glycine were also present. Accordingly, it was revealed that the oral intake of the collagen peptide composition of the present invention causes new peptides to enter the blood, which do not usually enter the blood. Furthermore, peptides in which the N-terminal amino acid was glycine were not detected among peptides in the blood collected after the oral intake of collagen peptide A or D. The results indicated that a tripeptide having the amino acid sequence of Gly-X-Y (which has been previously reported (see JP Patent Publication (Kokai) No. 2001-131084 A and JP Patent Publication (Kokai) No. 2003-137807 A) does not enter the blood in its intact form even when orally ingested, and thus it is ineffective. The reason that the tripeptide Gly-X-Y is not detected in the blood after the oral intake thereof may be that after oral intake, glycine is cleaved within the digestive tract and then only the remaining dipeptides enter the blood.

### (Example 4) Determination of the N-terminal amino acid residues of collagen peptide compositions

The N-terminal amino acid residues of the collagen peptide composition prepared in Example 1 and collagen peptides A to D used in Example 2 were examined. The amino acid sequences of the peptides contained in each collagen peptide were determined by dissolving each peptide in water, adding the solution dropwise onto a PVDF membrane, and then carrying out analysis using a protein sequencer (Applied Biosystems, an amino acid sequence analyzer with which the Edman method is carried out in an automated manner). Table 4 below shows the ratio of N-terminal glycine residues to total of the N-terminal amino acid residues of peptides composing each composition.

**Table 4**

| Collagen peptide composition | Ratio of N-terminal glycine residues to total of the N-terminal amino acid residues (mol%) |
|---|---|
| Collagen peptide composition of the present invention | 59. 4 |
| Collagen peptide A | 69. 0 |
| Collagen peptide B | 21. 6 |
| Collagen peptide C | 25. 3 |
| Collagen peptide D | 65. 4 |

As shown in Table 4, in the cases of collagen peptides A and D, the ratio of N-terminal glycine residues to total of the N-terminal amino acid residues was higher than 65 mol%. On the other hand, in the cases of collagen peptides B and C, the ratio of N-terminal glycine residues to total of the N-terminal amino acid residues was lower than 30 mol%. As described in Example 2, in the cases of these collagen peptides A to D, the ability to enter the blood of peptides was low. On the other hand, in the case of the collagen peptide composition of the present invention, the ratio of N-terminal glycine residues to total of the N-terminal amino acid residues was 59.4 mol% and good ability to enter the blood was observed. Hence, it is concluded that when the ratio of N-terminal glycine residues to total of the N-terminal amino acid residues of peptides composing the composition is within a proper range, the ability to enter the blood is high, and when the ratio is out of such range, the ability to enter the blood is lowered.

### (Example 5) Preparation of the collagen peptide compositions of the present invention

Various proteolytic enzymes were separately added to the gelatin solution obtained in Example 1, so that 5 types of powdery collagen peptide composition were obtained as the collagen peptide compositions of the present invention under the conditions of Table 5 below.

**Table 5**

| Collagen peptide composition of the present invention (name of sample) | Name and level of enzyme (per Kg of gelatin) | Reaction pH, reaction temperature, and reaction time | Deactivation conditions | Purification conditions |
|---|---|---|---|---|
| 02A | Subtilisin (6.0g) | pH8.0, | 90°C or | Addition of pulverized activated carbon 2.0% and gelatin followed by purification filtration |
| | | 50°C, | higher, | |
| | | 6 hours | 10 minutes | |
| 02B | Chymotrypsin | pH8.0, | Same as | Same as above |
| | (6.0 g) | 40°C, | above | |
| | | 6 hours | | |
| 02N | Actinidain (6.0 | pH7.5, | Same as | Same as above |
| | g) | 40°C, | | |
| | | 6 hours | above | |
| 02AF | Enzyme mixture | pH8.0, | Same as | Same as above |
| | of subtilisin | 40°C, | | |
| | (5.5g) and bromelain (3.0g) | 6 hours | above | |
| 02BF | Enzyme mixture | pH8.0, | Same as | Same as above |
| | of chymotrypsin | 40°C, | | |
| | (5.5g) and bromelain (3.0g) | 6 hours | above | |

The average molecular weights of the thus obtained collagen peptide compositions were examined by a method similar to that used in Example 1.

The ratio of N-terminal glycine residues to total of the N-terminal amino acid residues of peptides in each composition was examined by a method similar to that used in Example 4. Table 6 below shows the results. In addition, the average molecular weight of total of the collagen peptide compositions was 2000.

**Table 6**

| Collagen peptide composition of the present invention (name of sample) | Ratio of N-terminal glycine residues to total of the N-terminal amino acid residues (mol%) | Molecular weight of 500 or less (%) | Molecular weight of 500 or more to 3000 or less (%) | Molecular weight of 3000 or more (%) |
|---|---|---|---|---|
| 02A | 56.7 | 4.4 | 88.0 | 7.7 |
| 02B | 63.6 | 3.8 | 87.6 | 8.6 |
| 02N | 36.4 | 5.0 | 85.7 | 9.3 |
| 02AF | 58.9 | 4.0 | 87.1 | 8.9 |
| 02BF | 64.9 | 4.5 | 87.8 | 7.7 |

### (Example 6) Test for comparison of ability to enter the blood (absorption test (2))

Ability to enter the blood of the 5 types of collagen peptide composition of the present invention prepared in Example 5 was examined using guinea pigs. As a control, the collagen peptide D used in Example 2 was used. Male Hartley guinea pigs (7 weeks old) were used for the test. The dose of a test sample was 3 g/10 mL/kg body weight. The sample was dissolved in distilled water and then the solution was orally administered. Guinea pigs were fasted from the evening of the day before the test. Blood was collected over time from Guinea pigs under diethyl ether anaesthesia via the jugular vein before administration and at 0.5, 1, 2, and 6 hours after administration. Blood treatment and measurement of the level of hydroxyproline in blood plasma were carried out according to the method described in Example 2. Table 7 below shows the AUC₀₋₇ values (AUC: area under the curve of blood concentration of hydroxyproline-containing peptide-time) (hr • nmol/ml: mean value ± S.E.), as calculated for each blood plasma sample.

**Table 7**

| Blood hydroxyproline-containing peptide levels after oral administration of collagen peptide compositions | |
|---|---|
| Collagen peptide composition | Hydroxyproline-containing peptide level (hr • nmol/ml: mean value ± S.E.) |
| Collagen peptide D (Example 2) | 155.0 ± 12.5 |
| 02A | 247.4 ± 7.9** |
| 02B | 281.9 ± 20.6** |
| 02N | 264.5 ± 44.3 |
| 02AF | 269.7 ± 26.0** |
| 02BF | 257.2 ± 47.5 |

| | |
|---|---|
| **: p < 0.01 | |

Also in the case of the guinea pigs, the blood hydroxyproline-containing peptide levels reach a peak at 1 to 2 hours after intake. Table 8 below shows the level of hydroxyproline-containing peptide (nmol/ml: mean value ± S.E.) at 1 hour and the same at 2 hours after administration of each collagen peptide.

**Table 8**

| Collagen peptide composition | 1 hour later | 2 hours later |
|---|---|---|
| Collagen peptide D (Example 2) | 29.82 ± 5.13 | 27.84 ± 2.91 |
| 02A | 40.77 ± 4.90 | 54.75 ± 3.70** |
| 02B | 42.00 ± 5.72 | 66.04 ± 9.70* |
| 02N | 44.28 ± 5.84 | 58.24 ± 11.53 |
| 02AF | 42.16 ± 4.14 | 66.06 ± 10.36* |
| 02BF | 41.33 ± 10.48 | 61.55 ± 13.41 |

| | | |
|---|---|---|
| **: p < 0.01, *: p < 0.05 | | |

As shown in Table 7, when AUCs were compared, the ability to enter the blood of the collagen peptide compositions of the present invention had significantly increased compared with the cases of the commercially available collagen peptide D. Moreover, as shown in Table 8, the ability to enter the blood at 1 hour and the same at 2 hours after the intake of the collagen peptide compositions of the present invention had significantly increased compared with the case of the commercially available collagen peptide D.

### (Example 7) Test for comparison of ability to enter the blood (absorption test (3))

The ability to enter the blood of 2 types (02B and 02N) out of the 5 types of the collagen peptide composition of the present invention prepared in Example 5 was examined with human subjects. As a control, commercially available collagen peptide E (Nitta Gelatin Inc.; Trade name: IXOS HDL-50F; molecular weight of 500 or less (0.117%); molecular weight of 500 or more to 3000 or less (46.248%); and molecular weight of 3000 or more (53.637%); and the ratio of N-terminal glycine residues to total of the N-terminal amino acid residues: 72.1 mol%) was used.

The test conducted herein was a crossover test such that: the wash-out period of 6 or more days was set for 13 human volunteers; and each subject underwent a single instance of intake of the above 3 types of collagen peptide composition as test samples. Specifically, each subject was fasted for 12 hours, blood was collected before intake, and then the subject ingested each test sample. The intake level of each test sample was 25 g/65 kg body weight. At 0, 5, 1, 2, 4, and 7 hours after intake, 5 mL of blood was collected and then the level of hydroxyproline-containing peptide in blood plasma was found by a method similar to that in Example 2. Table 9 below shows the AUC₀₋₇ values (AUC:area under the curve of blood concentration of hydroxyproline-containing peptide-time) (hr • nmol/ml: mean value ± S.E.), as calculated for each blood plasma sample.

**Table 9**

| Blood hydroxyproline-containing peptide levels after the oral intake of collagen peptide compositions | |
|---|---|
| Collagen peptide composition | Hydroxyproline-containing peptide level (hr • nmol/ml: mean value ± S.E.) |
| Collagen peptide E | 225.8 ± 21.2 |
| 02B | 241.0 ± 19.4 |
| 02N | 255.1 ± 19.9** |

| | |
|---|---|
| **: p < 0.01 | |

The blood hydroxyproline-containing peptide levels reached peaks at 1 to 2 hours after intake. Table 10 below shows the hydroxyproline-containing peptide levels at 1 hour and the same at 2 hours after administration of each collagen peptide (nmol/ml: mean value ± S.E.)

**Table 10**

| Collagen peptide composition | 1 hour later | 2 hours later |
|---|---|---|
| Collagen peptide E | 63.5 ± 28.4 | 67.7 ± 30.1 |
| 02B | 76.6 ± 26.2 | 79.4 ± 30.6 |
| 02N | 82.5 ± 25.1** | 80.8 ± 24.6* |

| | | |
|---|---|---|
| **: p<0.01, *: p<0.05 | | |

As shown in table 9, when AUCs were compared, the ability to enter the blood of the collagen peptide compositions of the present invention was significantly increased compared with the same of the commercially available collagen peptide E. Moreover, as shown in Table 10, the ability to enter the blood at 1 hour and the same at 2 hours after the intake of the collagen peptide compositions of the present invention were significantly increased compared with the same of the commercially available collagen peptide E.

### Industrial Applicability

The present invention can be used in the fields of production of foods or beverages such as functional foods and supplements.

All publications, patents, and patent applications cited in this description are herein incorporated by reference in their entirety.

## Claims

1. A collagen peptide composition obtainable by digesting a collagen or gelatin with protease, which comprises 70% to 100% by weight of peptides with a molecular weight 500 or more to 3000 or less, less than 10% by weight of peptides with a molecular weight of less than 500, and less than 20% by weight of peptides with a molecular weight of more than 3000, based on the total weight of the composition, wherein the ratio of N-terminal glycine residues to total of the N-terminal amino acid residues of the peptides in the composition is 33 mol% or more to 65 mol% or less.

2. The collagen peptide composition according to claim 1, wherein the protease is serine protease or cysteine protease.

3. The collagen peptide composition according to claim 1 or 2, wherein the collagen or the gelatin is derived from fishes.

4. A food or beverage, which contains the collagen peptide composition according to any one of claims 1 to 3.
